# EUROPEAN PATENT APPLICATION

(11) **EP 4 817 532 A2**
(43) Date of publication of application: **07.10.2026**
(21) Application number: 26170355.7
(22) Date of filing: 01.04.2026
(51) Int. Cl.: A61B 17/02, A61B 17/70

(54) **VERTEBRAL RETRACTOR**

(30) Priority: 02.04.2025 US 202563782310 P
(71) Applicant: Shukla Medical, St. Petersburg, FL 33709 (US)
(72) Inventor: KISKA, Mitchell, Seminole, 33772 (US); AHMED, Osama, Bulverde, 78163 (US)
(74) Representative: Swea IP Law AB

(57) **Abstract**

A vertebral retractor including a first distraction pin having a threaded tip, a second distraction pin having a threaded tip, a first arm operatively connected to the first distraction pin for securing the first distraction pin in position, a second arm operatively connected to the second distraction pin for securing the second distraction pin in position, and an adjuster assembly operatively connected to the first and second arms for adjusting a relative position of the first and second arms.

## Description

### CROSS-REFERENCE TO RELATED APPLICATION

This application claims the benefit of priority to U.S. Patent Application No. 63/782,310, filed April 2, 2025, entitled "VERTEBRAL EXTRACTOR," the entire disclosure of which is hereby incorporated by reference for all purposes.

### FIELD OF THE INVENTION

The subject disclosure relates generally to surgical devices and, more particularly, to a vertebral retractor.

### BACKGROUND OF THE INVENTION

When performing a spinal surgical procedure to remove a spine cage implant from the spinal column, a surgeon must retract or create space between adjacent vertebrae to enable the spine cage implant to be removed. A vertebral retractor is used to separate adjacent vertebrae to enable removal of the spine cage implant.

### BRIEF SUMMARY OF THE INVENTION

According to a first exemplary embodiment of the subject disclosure, there is provided a vertebral retractor comprising a first distraction pin having a threaded tip, a second distraction pin having a threaded tip, a first arm operatively connected to the first distraction pin for securing the first distraction pin in position, a second arm operatively connected to the second distraction pin for securing the second distraction pin in position, and an adjuster assembly operatively connected to the first and second arms for adjusting a relative position of the first and second arms.

According to an aspect, each of the first and second distraction pins include a shaft, a self-tapping screw tip about a distal end of the shaft, a depth stop adjacent the self-tapping screw tip, and a quick connection about a proximal end opposite the self-tapping screw tip.

According to an aspect, each of the first and second arms include an elongated body, and a distal end configured for operative engagement with a respective first or second distraction pin. According to another aspect, the elongated body is a non-linear body.

According to an aspect, the adjuster assembly comprises a body operatively connected to each of the first and second arms, and an adjuster extending from the body and operatively connected to each of the first and second arms for adjusting the relative position of the first and second arms. According to another aspect, the adjuster assembly comprises an adjuster, and a linkage assembly operatively connected to the adjuster and linking each of the first and second arms for adjusting the relative position of the first and second arms. According to another aspect, the linkage assembly comprises a main body linkage connected to each of the first and second arms, a first pivot linkage operatively connected to the first arm and the adjuster, and a second pivot linkage operatively connected to the second arm and the adjuster. According to another aspect, the adjuster comprises a threaded body, a collar rotatably receiving the threaded body, and a fastener for securing the collar about a fixed position on the threaded body.

According to the above aspects, a turning of the threaded body in a first direction causes the threaded body and the collar to move distally relative to the main body linkage. Distal movement of the collar relative to the main body linkage causes rotational movement of each of the first and the second pivot linkages relative to the collar. This causes rotational movement of each of the first and the second arms relative to the first and the second pivot linkages, therein causing separation of the first and the second arms, which results in separation of the first and the second vertebra engaging distraction pins.

According to a further exemplary embodiment of the subject disclosure, there is provided a vertebral retractor comprising a crossbar, a first arm having a first end pivotably connected to the crossbar and second end including an opening, a second arm having a first end pivotably connected to the crossbar and second end including an opening, a pair of vertebra engaging pins each slidably received in one of the openings of the first and second arms, and an adjuster assembly operably connected to the first and second arms for adjusting spacing between the first and second arms.

According to an aspect, each of the pair of vertebra engaging pins has a proximal end configured for turning by a tool and a distal end for engaging a vertebra. According to another aspect, the distal end of each vertebra engaging pin is a screw configured for threading into a vertebra. According to another aspect, the screw is self-tapping. According to another aspect, each of the pair of vertebra engaging pins includes a shoulder for limiting penetration of the screw into a vertebra. According to another aspect, each of the pair of vertebra engaging pins has a length of about 140 mm to 200 mm.

According to an aspect, the adjuster assembly comprises a screw threadedly received in the crossbar. According to another aspect, a proximal end of the screw includes a knob, whereby turning of the knob in a first direction enlarges the spacing between the first and second arms and turning of the knob in a second direction opposite the first direction reduces the spacing between the first and second arms.

According to an aspect, a proximal end of the screw includes a knob, and the adjuster assembly further comprises a collar rotatably receiving the screw. In this aspect, turning of the knob in a first direction causes the threaded screw and the collar to each move distally relative to the crossbar, resulting in an increase of space between the first and the second arms and an increase in space between the pair of vertebra engaging pins. Further, turning of the knob in a second direction, opposite the first direction, causes the threaded screw and the collar to each move proximally relative to the crossbar, resulting in a reduction in spacing between the first and the second arms and a reduction in spacing between the pair of vertebra engaging pins.

According to another aspect, the adjuster assembly further comprises a collar rotatably receiving the screw, a first link pivotably connecting the collar to the first arm, and a second link pivotably connecting the collar to the second arm. In this aspect, turning of the screw in a first direction causes the screw and the collar to move distally relative to the crossbar. This distal movement of the collar relative to the crossbar causes rotational movement of each of the first and the second links relative to the collar. This results in rotational movement of each of the first and the second arms relative to the first and the second links, causing separation of the first and the second arms, therein causing separation of the first and the second vertebra engaging distraction pins. In still another aspect, the vertebral retractor further comprises a nut secured to a distal end of the screw for retaining the collar about the screw.

According to an aspect, the first and second arms each include a proximal portion and a distal portion disposed at an obtuse angle relative to the proximal portion. According to another aspect, the distal portions of the first and second arms diverge from each other.

### BRIEF DESCRIPTION OF THE SEVERAL VIEWS OF THE DRAWINGS

The foregoing summary, as well as the following detailed description of the exemplary embodiments of the invention, will be better understood when read in conjunction with the appended drawings. For the purpose of illustrating the invention, there are shown in the drawings embodiments, which are presently preferred. It should be understood, however, that the invention is not limited to the precise arrangements and instrumentalities shown.

In the drawings:
FIG. 1 is a perspective view of an exemplary embodiment of a vertebral retractor of the subject disclosure;
FIGs. 1A and 1B are elevation views of the vertebral retractor of FIG. 1 in first and second positions, respectively;
FIG. 2 is another elevation view of the vertebral retractor of FIG. 1;
FIG. 3 is a perspective view of a body, main body linkage or crossbar of the vertebral retractor of FIG. 1;
FIG. 4 is a perspective view of an arm of the vertebral retractor of FIG. 1;
FIG. 5 is a top plan view of the arm of FIG. 4;
FIG. 6 is an elevation view of a vertebra engaging distraction pin of the vertebral retractor of FIG. 1;
FIG. 7 is a perspective view of a threaded body or screw of an adjuster assembly of the vertebral retractor of FIG. 1;
FIG. 8 is a perspective view of a collar of an adjuster assembly of the vertebral retractor of FIG. 1;
FIG. 9 is a perspective view of a pivot linkage or link of an adjuster assembly of the vertebral retractor of FIG. 1; and
FIG. 10 is a fastener or nut of an adjuster assembly of the vertebral retractor of FIG. 1.

### DETAILED DESCRIPTION

Reference will now be made in detail to the various embodiments of the subject disclosure illustrated in the accompanying drawings. Wherever possible, the same or like reference numbers will be used throughout the drawings to refer to the same or like features. It should be noted that the drawings are in simplified form and are not drawn to precise scale. Certain terminology is used in the following description for convenience only and is not limiting. Directional terms such as top, bottom, left, right, above, below and diagonal, are used with respect to the accompanying drawings. The term "distal" shall mean away from the center of a body. The term "proximal" shall mean closer towards the center of a body and/or away from the "distal" end. The words "inwardly" and "outwardly" refer to directions toward and away from, respectively, the geometric center of the identified element and designated parts thereof. Such directional terms used in conjunction with the following description of the drawings should not be construed to limit the scope of the subject disclosure in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

"Substantially" as used herein shall mean considerable in extent, largely but not wholly that which is specified, or an appropriate variation therefrom as is acceptable within the field of art. "Exemplary" as used herein shall mean serving as an example.

Throughout this disclosure, various aspects of the subject disclosure can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments. One skilled in the relevant art will recognize, in light of the description herein, that the present disclosure can be practiced without one or more of the specific features or advantages of a particular exemplary embodiment. In other instances, additional features and advantages may be recognized in certain embodiments that may not be present in all exemplary embodiments of the subject disclosure.

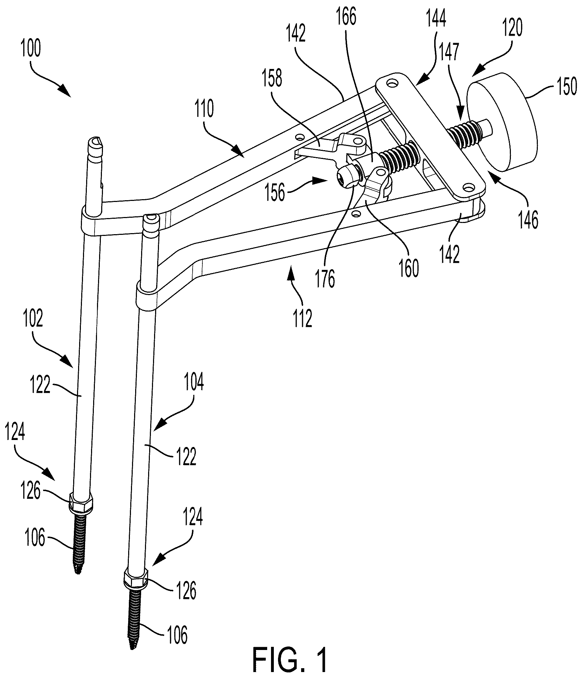

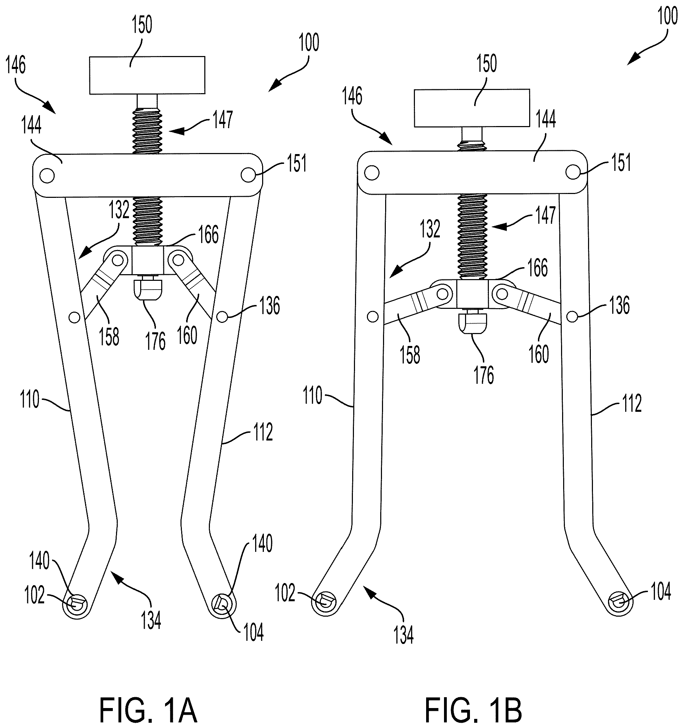

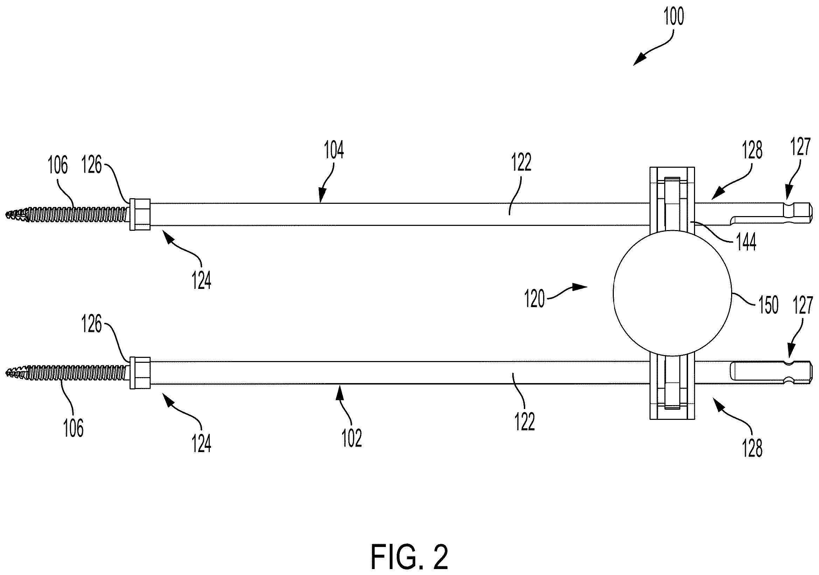

Referring to FIGS. 1 and 2, there is shown a vertebral retractor 100 constructed in accordance with an exemplary embodiment of the subject disclosure. The vertebral retractor 100 comprises a pair of (i.e., first and second) vertebra engaging distraction pins 102, 104, each of which has a threaded tip 106. The vertebral retractor 100 further comprises a first arm 110, operatively connected (slidably connected) to the first distraction pin 102, for securing the first distraction pin 102 in position; and a second arm 112, operatively connected (slidably connected) to the second distraction pin 104, for securing the second distraction pin 104 in position. In addition, the vertebral retractor 100 comprises an adjuster assembly 120 operatively connected (rotatably connected) to the first and to the second arms 110, 112 for adjusting a relative position of, or spacing between, the first and second arms 110, 112.

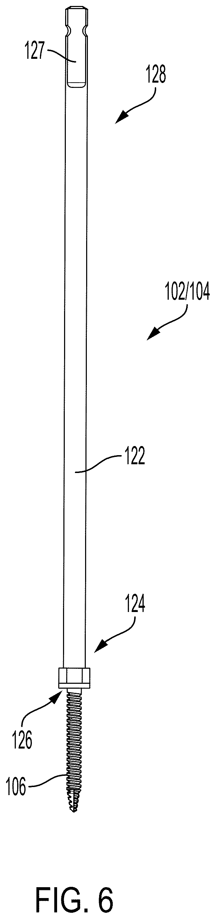

Referring to FIGS. 2 and 6, each of the first and the second vertebra engaging distraction pins 102, 104 has a length of about 140 mm to 200 mm. The distraction pins 102, 104 each include a shaft 122, and the threaded tip 106 (which is preferably a self-tapping screw or screw tip) about a distal end 124 of the shaft 122 for threading into a vertebra. Also included is a depth stop or shoulder 126, adjacent the self-tapping screw/threaded tip 106, for limiting penetration of the screw tip into a vertebra. The self-tapping screw/threaded tip 106 might be quite lengthy, and extend a substantial portion of the distraction pin 102, 104 length, to allow for larger patients. Also included is a quick connection 127 about a proximal end 128 of each distraction pin 102, 104 (the end opposite the self-tapping screw tip 106) which is configured for turning by a tool having a mating quick connection. For example the quick connection 127 can be a AO connection end (i.e., AO Quick Coupling or AO Fitting), which is a standardized, D-shaped (flattened side) with circular groove, or hex-shaped shank, on the proximal end of surgical drill bits, reamers, and drivers, designed for quick, blind insertion, and lockedly secure attachment to orthopedic power tools or hand handles, preventing slipping during possibly high-torque bone drilling..

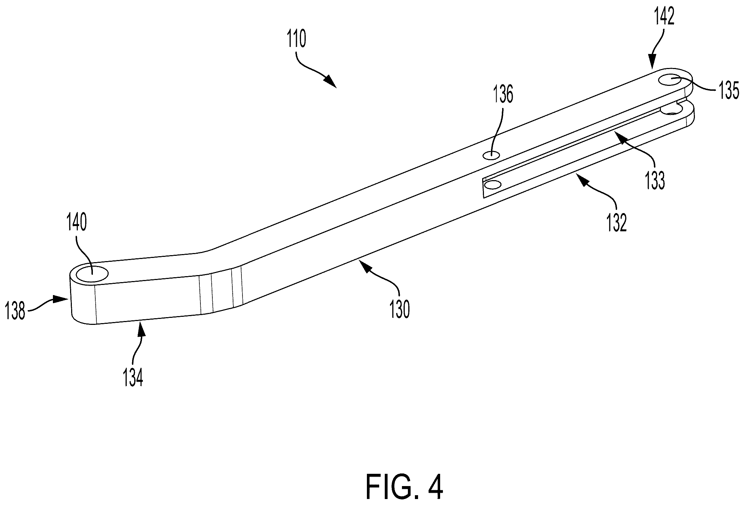

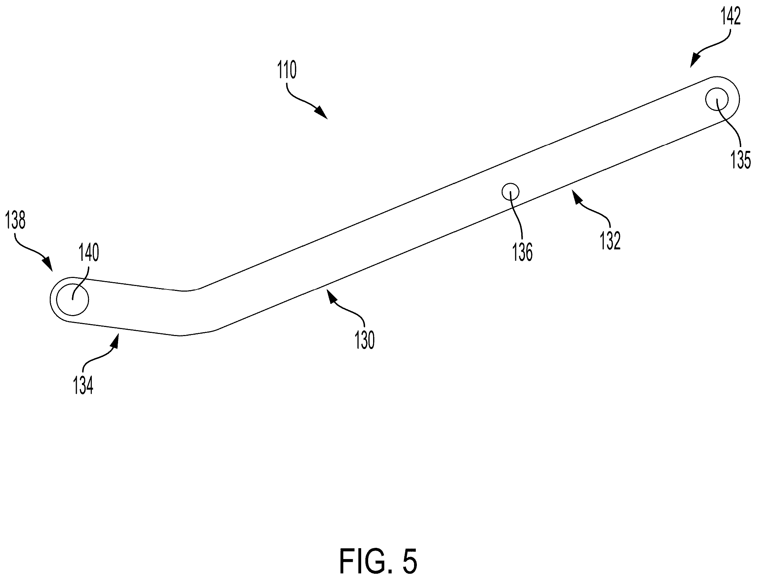

Referring to FIGS. 4 and 5, there is shown an exemplary construction of the first arm 110, wherein the construction of the second arm 112 is a mirror image thereof. Each of the first and the second arms 110, 112 comprise a non-linear elongated body 130 including a proximal portion 132 and a distal portion 134. The distal portion 134 is disposed at an angle, preferably an obtuse angle (in one aspect, between 135° and 160°), relative to the proximal portion, whereby the distal portions 134 of the first and the second arms 110, 112 diverge away from each other, as best shown in FIG. 1. A second or distal end 138 of the distal portion 134 includes an opening 140 configured for operative engagement with a respective first or second distraction pin 102, 104. More particularly, a respective one of each of the first and the second distraction pins 102, 104 is slidably received in a respective one of the openings 140 of the first and the second arms 110, 112. In one aspect, the diametrically circular opening 140 simply slidably receives a diametrically circular, cylindrical portion of the respective first or second distraction pin 102, 104, without securable, or releasably lockable, engagement. In this aspect, a weight of the vertebral retractor 100 can tend to hold the retractor 100 in place. Further, this slidable relationship between the members allows for positioning the retractor 100 (i.e., arm 110, 112) at any point along the respective pin (102, 104) to account for varying patient size and quick attachment/release of the retractor 100 from the pins 102, 104.

A first or proximal end 142 of each of the first and the second arms 110, 112 is operatively (e.g., pivotably) connected (via aligned openings 135 and an unillustrated pin) to a body 144 (also referred to a main body linkage or a crossbar) of the adjuster assembly 120 (as described below). In one aspect, the proximal portion of each arm 110, 112 can include a rectangular recess 133 therein, on a medial side thereof, to accommodate rotatable connection to, and movement of, an operatively engaging a first or a second pivot linkage 158, 160 (as described below). Aligned openings 136 provide for pivotal connection (via unillustrated pin spanning the recess 133) of a first, distal end 161 of the first or the second pivot linkage 158, 160 to the respective first or second arm 110, 112.

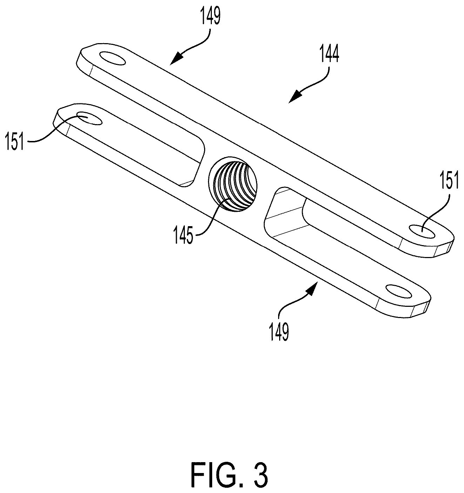

FIGS. 1-3 and 7-10 collectively illustrate an exemplary embodiment of the adjuster assembly 120. According to an aspect, the adjuster assembly 120 includes the body/main body linkage/crossbar 144 (FIG. 3) that includes a central threaded opening 145 with two bifurcated extensions 149, each pair of extensions 149 radially extending from the central threaded opening 145. At a distal end of each individual extension 149 is an opening 151, aligned with opening 151 of the accompanying individual extension 149 of the bifurcated pair. A longitudinal axis of the central threaded opening 145 is transverse a longitudinal axis of all openings 151 of the two bifurcated extensions 149.

A proximal end 142 of each of the first and the second arms 110, 112 is inserted within a bifurcated extension 149 of the crossbar 144, and with openings 135 and holes 151 aligned, the respective members are rotatably connected via unillustrated pin to operatively (e.g., pivotably) connect each of the first and the second arms 110, 112 to the body/crossbar 144.

The adjuster assembly 120 also includes an adjuster 146 extending from the body 144 and operatively connected (rotatably connected) to each of the first and the second arms 110, 112 for adjusting a relative position of the first and the second arms 110, 112, and thereby a relative position of the first and the second vertebra engaging distraction pins 102, 104.

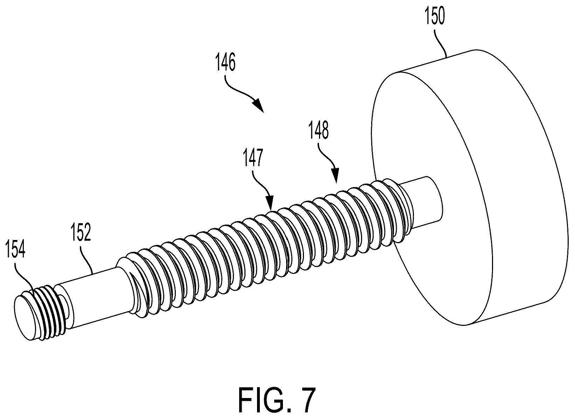

As shown in FIG. 7, the adjuster 146 comprises a threaded body or screw 147 including a proximal threaded portion 148 threadedly received in the central threaded opening 145 of the crossbar 144. The adjuster 146 also includes a knob 150, where a turning of the knob 150 in a first direction enlarges a spacing between the first and the second arms 110, 112, and a turning of the knob 150 in a second direction (opposite the first direction) reduces the spacing between the first and the second arms 110, 112. The threaded body 147 of the adjuster 146 further includes a smooth cylindrical intermediate portion 152 (contiguous with both the proximal threaded portion 148 and a distal threaded portion 154). Accordingly, operative turning of the knob 150 can lengthen, shorten, or hold a distance between the first and the second vertebra engaging distraction pins 102, 104, facilitating surgeon access to a spine cage of a patient.

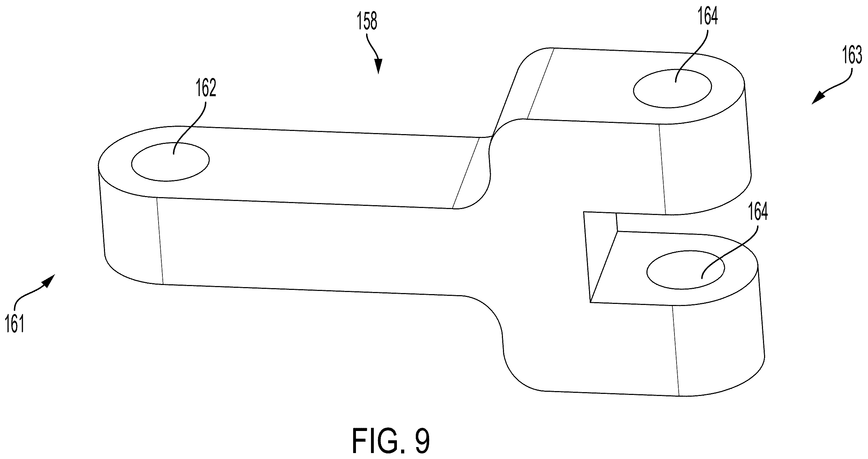

The adjuster assembly 120 includes a linkage assembly 156 (FIG. 1) operatively connected (fixedly, threadably, and/or pivotably connected) to the adjuster 146 and operatively linking each of the first and the second arms 110, 112 to adjust a relative position of the first and the second arms 110, 112. The linkage assembly 156 comprises the main body linkage or crossbar 144 (which is pivotably connected to each of the first and the second arms 110, 112); a first link or first pivot linkage 158 (FIGS. 1 and 9) operatively connected (pivotably connected) to the first arm 110 and to the adjuster 146, and a second link or second pivot linkage 160 operatively connected (pivotably connected) to the second arm 112 and to the adjuster 146.

An exemplary construction of the first link 158 is shown in FIG. 9, where the construction of the second link 160 is a mirror image thereof. A first (or distal) end 161 of the first link 158 includes an opening 162 for receiving an unillustrated pivot pin for pivotably connecting the first link 158 to an intermediate portion of the first arm 110 (distal end 161 of the first link 158 is received within recess 133, where opening 162 of the first link 158 aligns with openings 136 of the first arm 110 for reception of the unillustrated pivot pin). A bifurcated second (or proximal) end 163 of the first link 158 includes a pair of aligned openings 164 for reception therebetween of a wing 172 of a collar 166 (discussed below). A longitudinal axis of the opening 162 at the distal end 161 is parallel to a longitudinal axis of the openings 164 in the bifurcated proximal end 163.

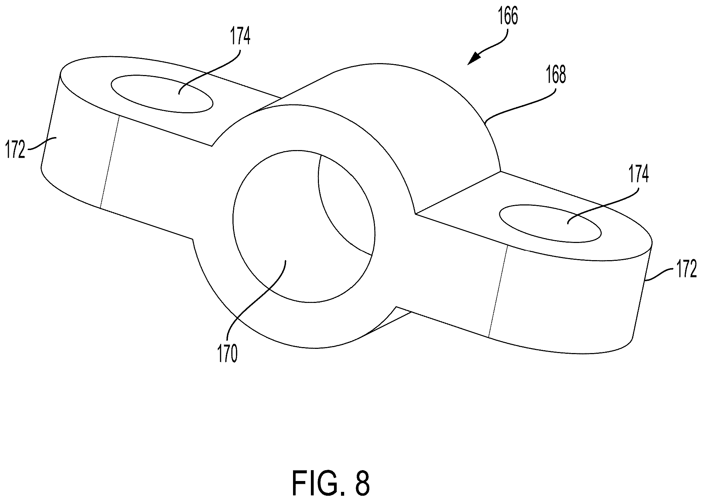

Referring to FIG. 8, there is shown a further component of the adjuster 146. FIG. 8 illustrates an exemplary collar 166, that includes a cylindrical central portion 168 having a through opening 170 for rotatably receiving the smooth cylindrical intermediate portion 152 of the threaded body 147 of the adjuster 146. At opposite sides of the cylindrical central portion 168, the collar 166 is provided with two radially extending wings 172 (each of which includes an opening 174). A longitudinal axis of each opening 174 of each wing 172 is transverse a longitudinal axis of through opening 170 of the central portion 168. Each wing 172 is received within a respective bifurcated second end 163 of the first and the second pivot linkage 158, 160; whereby the aligned openings 164 of each bifurcated second end 163 aligns with a respective opening 174 of each wing 172 to receive an unillustrated pivot pin for pivotably connecting the first and the second links 158, 160 to the collar 166.

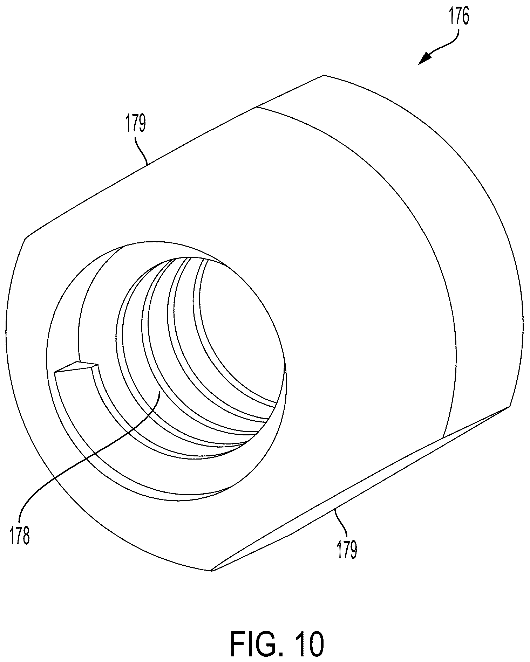

Figs 1 and 10 show a further component of the adjuster 146, namely, a fastener or nut 176 having a threaded opening 178 configured to threadedly receive the distal threaded portion 154 of the adjuster 146, to secure or retain the collar 166 at or about a fixed position on the threaded body 147 of the adjuster 146. Note that the fastener 176 has relatively rounded edges/sides, but can include flattened side portions 179 to accommodate the close-in location of the fastener 176 between the first and the second arms 110, 112, and to facilitate easy fastening of the fastener on the distal threaded portion 154 of the adjuster 146.

In operation of the vertebral retractor 100, a turning of the knob 150 of the adjuster 146 in a first direction causes the threaded body/screw 147 to move distally (relative to a stationary body/crossbar 144) and to move the collar 166 distally (relative to a stationary body/crossbar 144) from a first position (FIG. 1A) to a second position (FIG. 1B). Distal movement of the collar 166 causes rotational movement of each of the first and the second pivot linkages 158, 160 relative to the collar 166, thereby causing rotational movement of each of the first and the second arms 110, 112 relative to the first and the second pivot linkages 158, 160. This rotational movement, initiated by the distal movement of the threaded body/screw 147 of the adjuster 146, acts to separate the first and the second arms 110, 112 (FIG. 1B), thereby separating the first and the second vertebra engaging distraction pins 102, 104. With the threaded, self-tapping screw tip 106 of each distraction pin 102, 104 implanted/engaged with vertebral bone, and spanning a vertebral gap, the separating distraction pins 102, 104 will widen the vertebral gap, allowing a surgeon access to the widened vertebral gap (e.g., to remove a spine cage implant).

The arrangement of the vertebral retractor 100 components, when the first and the second vertebra engaging distraction pins 102, 104 are near (or fully) separated (i.e., where the first and the second pivot linkages 158, 160 are fully extended (see FIG 1B)) provide a large enough open space therebetween to accommodate surgeon viewing of, or other surgical tools/instruments into, the widened vertebral gap.

It will be appreciated by those skilled in the art that changes could be made to the various aspects described above without departing from the broad inventive concept thereof. It is to be understood, therefore, that the subject application is not limited to the particular aspects disclosed, but it is intended to cover modifications within the spirit and scope of the subject application as defined by the appended claims.

## Claims

1. A vertebral retractor (100) **characterized in that**, and comprising:
a first distraction pin (102) having a threaded tip;
a second distraction pin (104) having a threaded tip;
a first arm (110) operatively connected to the first distraction pin (102) for securing the first distraction pin (102) in position;
a second arm (112) operatively connected to the second distraction pin (104) for securing the second distraction pin (104) in position; and
an adjuster assembly (120) operatively connected to the first and the second arms (110, 112), the adjuster assembly (120) being operable to adjust a relative position of the first and the second arms (110, 112).

2. The vertebral retractor (100) of claim 1, wherein each of the first and the second distraction pins (102, 104) include:
a shaft (122);
a self-tapping screw tip (106) about a distal end (124) of the shaft (122), the self-tapping screw tip (106) comprising the threaded tip;
a depth stop (126) adjacent the self-tapping screw tip (106); and
a quick connection (127) about a proximal end (128) opposite the self-tapping screw tip (106).

3. The vertebral retractor (100) of any preceding claim, wherein each of the first and the second arms (110, 112) include:
a non-linear elongated body (130); and
a distal end (138) configured for operative engagement with a respective first or second distraction pin (102, 104).

4. The vertebral retractor (100) of any preceding claim, wherein the adjuster assembly (120) comprises:
a body (144) operatively connected to each of the first and the second arms (110, 112); and
an adjuster (146) extending from the body (144) and operatively connected to each of the first and the second arms (110, 112) for adjusting the relative position of the first and the second arms (110, 112).

5. The vertebral retractor (100) of any of claims 1-3, wherein the adjuster (146) assembly (120) comprises:
an adjuster (146); and
a linkage assembly (156) operatively connected to the adjuster (146) and linking each of the first and the second arms (110, 112) for adjusting the relative position of the first and the second arms (110, 112).

6. The vertebral retractor (100) of claim 5, wherein the linkage assembly (156) comprises:
a main body linkage (144) connected to each of the first and the second arms (110, 112);
a first pivot linkage (158) operatively connected to the first arm (110) and the adjuster (146); and
a second pivot linkage (160) operatively connected to the second arm (112) and the adjuster (146).

7. The vertebral retractor (100) of claim 5, wherein the adjuster (146) comprises:
a threaded body;
a collar (166) rotatably receiving the threaded body (147); and
a fastener (176) for securing the collar (166) about a fixed position relative to the threaded body (147).

8. The vertebral retractor (100) of claim 5, wherein the adjuster (146) comprises:
a threaded body (147); and
a collar (166) rotatably receiving the threaded body (147);
wherein the linkage assembly (156) comprises:
a main body linkage (144) connected to each of the first and second arms (110, 112);
a first pivot linkage (158) operatively connected to the first arm (110) and to the collar (166); and
a second pivot linkage (160) operatively connected to the second arm (112) and to the collar (166);
wherein:
a turning of the threaded body (147) in a first direction causes the threaded body (147) and the collar (166) to move distally relative to the main body linkage (144); and
distal movement of the collar (166) relative to the main body linkage (144) causes rotational movement of each of the first and the second pivot linkages (158, 160) relative to the collar (166), therein causing rotational movement of each of the first and the second arms (110, 112) relative to the first and the second pivot linkages (158, 160); therein causing separation of the first and the second arms (110, 112), therein causing separation of the first and the second distraction pins (102, 104).

9. The vertebral retractor (100) of claim 1, wherein:
the adjuster assembly (120) includes a crossbar (144);
the first arm (110) includes a first end pivotably connected to the crossbar (144) and a second end including an opening (140);
the second arm (112) includes a first end pivotably connected to the crossbar (144) and a second end including an opening (140); and
the first and the second distraction pins (102, 104) are each slidably received in one of the openings (140) of the first and the second arms (110, 112);
the adjuster assembly (120) is operably connected to the first and the second arms (110, 112) to adjust a spacing between the first and the second distraction pins (102, 104).

10. The vertebral retractor (100) of claim 9, wherein each of the first and the second distraction pins (102, 104) has a proximal end (128) configured for turning by a tool and a distal end (124) including a screw (106) configured for threading into a vertebra.

11. The vertebral retractor (100) of claim 10, wherein each of the first and the second distraction pins (102, 104) includes a shoulder (126) for limiting penetration of the screw (106) into a vertebra.

12. The vertebral retractor (100) of claim 9, wherein the adjuster assembly (120) further comprises a screw (147) threadedly received in the crossbar (144).

13. The vertebral retractor (100) of claim 12, where:
a proximal end (148) of the screw (147) includes a knob (150);
the adjuster assembly (120) further comprises a collar (166) rotatably receiving the screw (147);
a turning of the knob (150) in a first direction causes the threaded screw (147) and the collar (166) to each move distally relative to the crossbar (144), resulting in an increase of space between the first and the second arms (110, 112) and an increase in space between the pair of distraction pins (102, 104); and
a turning of the knob (150) in a second direction, opposite the first direction, causes the threaded screw (147) and the collar (166) to each move proximally relative to the crossbar (144), resulting in a reduction in spacing between the first and the second arms (110, 112) and a reduction in spacing between the pair of distraction pins (102, 104).

14. The vertebral retractor (100) of claim 12, where the adjuster assembly (120) further comprises:
a collar (166) rotatably receiving the screw (147);
a first link (158) pivotably connecting the collar (166) to the first arm (110); and
a second link (160) pivotably connecting the collar (166) to the second arm (112);
where:
a turning of the screw (147) in a first direction causes the screw (147) and the collar (166) to move distally relative to the crossbar (144);
distal movement of the collar (166) relative to the crossbar (144) causes rotational movement of each of the first and the second links (158, 160) relative to the collar (166), therein causing rotational movement of each of the first and the second arms (110, 112) relative to the first and the second links (158, 160); therein causing separation of the first and the second arms (110, 112), therein causing separation of the first and the second distraction pins (102, 104).

15. The vertebral retractor (100) of claim 9, wherein the first and second arms (110, 112) each include a proximal portion (132) and a distal portion (134), where the distal portion (134) is disposed at an obtuse angle relative to the proximal portion (132); and where the angled distal portions (134) diverge away from each other.
